# EUROPÄISCHE PATENTSCHRIFT

(11) **EP 1 658 021 B1**
(45) Veröffentlichungstag und Bekanntmachung des Hinweises auf die Patenterteilung: **20.04.2011**
(21) Anmeldenummer: 04764477.8
(22) Anmeldetag: 25.08.2004
(51) Int. Cl.: A61F 2/16

(54) **CILIARMUSKEL-BETÄTIGTES, AKKOMODATIONSFÄHIGES LINSENIMPLANTAT**
ACCOMMODATIVE LENS IMPLANT, CONTROLLED BY THE CILIARY MUSCLE
IMPLANT CRISTALLINIEN ACTIONNE PAR LE MUSCLE CILIAIRE ET APTE A L'ACCOMMODATION

(30) Priorität: 26.08.2003 DE 10339266; 02.10.2003 DE 10346024
(43) Veröffentlichungstag der Anmeldung: 24.05.2006
(73) Patentinhaber: Carl Zeiss Meditec AG, 07745 Jena (DE)
(72) Erfinder: SCHEDLER, Markus, 81479 München (DE)
(74) Vertreter: Kruspig, Volkmar
(86) Internationale Anmeldenummer: PCT/EP2004/009501
(87) Internationale Veröffentlichungsnummer: WO 2005/020857

(56) Entgegenhaltungen:
- EP-A- 0 356 050
- US-A- 4 731 078
- US-A- 5 443 506
- US-A- 5 800 533
- US-A- 6 117 171

## Beschreibung

Die Erfindung betrifft ein Ciliarmuskel-betätigtes, akkommodationsfähiges Linsenimplantat, bestehend aus mindestens einer oder mehreren auf einer gemeinsamen optischen Achse liegenden Linsen aus einem bevorzugt biokompatiblen Material.

Aus der EP 0 793 460 B1 ist ein akkommodierendes künstliches Linsenimplantat vorbekannt, welches Optiken verwendet, die sich als Reaktion auf Veränderungen im Ciliarmuskel bewegen. Die dortige intraokulare Linsenanordnung soll in ein menschliches Auge implantierbar sein, wobei das Auge zumindest einen Abschnitt einer Linsenkapsel, einen Ciliarmuskel und vom Ciliarmuskel gesteuerte Zonulae umfasst. Über einen speziellen Ring, der so ausgelegt ist, um mit dem Ciliarmuskel zusammenzuwirken, werden Hebelarme gebildet, deren jeder an einer ersten Position des Hebelarms an der Optik gelenkig angebracht ist und an einer zweiten Position des Hebelarms an dem vorerwähnten Ring fixiert wird. Die Hebelarme sollen eine Verstärkung bzw. Vergrößerung der möglichen Linsenbewegungen erzeugen.

Adaptive Linsen, deren Abstand entlang der optischen Achse zur Verbesserung des Akkommodationsvermögens angeordnet sind, werden beispielsweise in dem US-Patent 5,275,623 beschrieben. Konkret wird dort das Fokussieren durch axiales Verschieben der Linse in Reaktion auf die normale Kontraktion und Expansion des Ciliarmuskels erreicht. Bei der Lösung nach US-PS 5,275,623 bleibt die Linsenkapsel vorhanden, jedoch wird mindestens eine zusätzliche Linse eingesetzt. Der dann wieder geschlossene Hohlkörper, der im dort erwähnten Ausführungsbeispiel zwei axial beabstandete Linsen aufnimmt, kann nach der Linsenimplantation mit einem Gas oder einer Flüssigkeit gefüllt werden, um einen Grundabstand der beiden Linsen zu erreichen.

Eine derartige Implantation ist wenig erfolgversprechend, da sich die Linsenkapsel mit heutigen Mitteln praktisch nicht chirurgisch verschließen lässt. Somit fehlt ein entscheidendes Element zur Einleitung der Muskelkräfte in das Implantat.

Der vorstehend beschriebene Stand der Technik ermöglicht außerdem keine chirurgische Grundeinstellung, um das intraokulare Linsenimplantat präzise im korrekten Abstand zur Netzhaut anzubringen, um eine gewünschte optimale Fern- und Nahsicht zu gestatten.

Aus der DE 689 02 614 T2 ist eine akkomodierende intraokulare Linse für das menschliche Auge vorbekannt, welche sich als Reaktion auf die Bewegung des Augenmuskels im Lichtbrechungsvermögen ändert, um Gegenstände scharf einzuistellen, die sich, vom Betrachter aus gesehen in verschiedenen Entfernungen befinden. Diesbezüglich ist dort eine Fluidkammer, welche unter Druck steht, vorhanden, wobei eine Stützblase erforderlich ist, die die Linsenkapsel so weit dehnt, dass der Ciliarkörper berührt wird. Problematisch ist bei der Lösung nach DE 689 02 614 T2 die Verankerung des Implantats in der zarten und empfindlichen Linsenkapsel mit einem entsprechenden Risiko der Zerstörung dieser vor, während oder nach der Implantation.

Auch bei dem intraokularen Linsenimplantat nach US 6,217,612 B1 erfolgt eine Verankerung in der Linsenkapsel mit all den erwähnten Schwierigkeiten während der Operation und postoperativ.

Gemäß der Lehre nach US 4,892,543 kommt ein Fluidkissen zum Einsatz, das durch die Kontur einer starren Linse in eine konvexe Form gedrückt wird. Dieses Fluidkissen kann entweder auf den Ciliarmuskel angreifen oder in die Linsenkapsel eingesetzt werden.

Bei der künstlichen Linsenanordnung nach US 4,888,012 wird ein Fluiddepot eingesetzt, welches eine durch Befüllung gebildete Linse steuert. Die Lichtbrechung erfolgt dort durch die optischen Eigenschaften des Fluids, was bedeutet, dass eine andere Substanz als die natürlich vorhandene eingesetzt werden muss.

In der EP 0 356 050 A1 wird eine intraokulare Vorrichtung beschrieben, welche eine innere und eine äußere Linse umfasst, wobei diese Linsen eine fluidgefüllte Kammer definieren. Des Weiteren ist eine Akkomodationsvorrichtung zum Verändern der Form der Kammer umfasst. Diese Vorrichtung weist eine Fluideinrichtung auf, welche zum selektiven Ändern des Fluiddrucks der Kammer ausgebildet ist. Aufgrund des sich ändernden Fluiddrucks wird die Position der äußeren Linse bezüglich der inneren Linse verändert. Des Weiteren ist eine Blase vorgesehen, welche für einen Kontakt mit den Augenmuskeln ausgebildet ist und mit der fluidgefüllten Kammer in Bewegung steht, wobei die Blase als Reaktion auf die Bewegung des Augenmuskel kontrahierbar und extrahierbar ist, sodass der Fluiddruck und letztendlich die Form der Kammer abhängig von der Augenmuskelbewegung verändert wird.

Aus dem Vorgenannten ist es daher Aufgabe der Erfindung, ein weiterentwickeltes Ciliarmuskel-betätigtes, akkommodationsfähiges Linsenimplantat, bestehend aus mindestens einer oder mehreren auf einer gemeinsamen optischen Achse liegenden Linsen anzugeben, welches geeignet ist, die natürliche Augenlinse zu ersetzen, und wobei die Möglichkeit besteht, auch nachträglich eine Einstellung vorzunehmen, um eine natürliche Akkommodation zu erreichen, so dass das Auge auch ohne Korrektionsbrille korrekt fokussieren kann, wobei des Weiteren eine Möglichkeit geschaffen werden soll eine nachträgliche postoperative Grundeinstellung der Position der mindestens einen Linse vornehmen zu können.

Die Lösung der Aufgabe der Erfindung erfolgt mit einem Ciliarmuskel-betätigten, akkommodationsfähigen Linsenimplantat gemäß den Merkmalen des Patentanspruchs 1, wobei die Unteransprüche eine mindestens zweckmäßige Ausgestaltung und Weiterbildung darstellen.

Gemäß dem Grundgedanken der Erfindung folgend, wird bei einer ersten Ausführungsform von einem vollständigen Verdrängen der ursprünglichen, natürlichen Augenlinse bzw. Linsenkapsel ausgegangen.

Das Linsenimplantat besitzt einen flexiblen, geschlossenen, im Bereich der eigentlichen Linse transparenten Implantatkörper, welcher im Sulcus der hinteren Augenkammer eingesetzt und fixiert wird, bzw. einen Implantatkörper, welcher großflächig am Ciliarmuskel aufgesetzt wird. Der Implantatkörper umfasst mindestens eine auf der Sehachse angeordnete Linse.

Der Implantatkörper mit der oder den Linsen weist eine Fluidfüllung auf, deren Volumen veränderbar ist.

Die axiale Position der Linse oder bei mehreren Linsen des sich ergebenden Linsensystems ist über ein Aktivieren der Ciliarmuskeln veränderbar, so dass das gewünschte Fokussieren zum Zweck der Nah- und Fernsicht gewährleistet ist.

Die notwendige Formveränderung des Implantatkörpers zum Zweck der Veränderung der Position der dortigen Linse längs der optischen Achse des Auges wird durch den Halt auf dem Ciliarmuskel und die Möglichkeit der Kontraktion, aber auch der Ausdehnung mit Blick auf den aktivierten Ciliarmuskel erreicht. Die Verankerung kann beispielsweise im Sulcus der Camera posterior erfolgen.

Um nun die gewünschte Einstellung der optischen Grundposition unter Beachtung der natürlichen Gegebenheiten des jeweiligen Patienten zu erreichen, steht der Fluidfüllungshohlraum des Implantatkörpers erfindungsgemäß mit einem, vorzugsweise im vorderen Teil des Bulbus oculi befindlichen Fluiddepots in Verbindung.

Das Fluiddepot ist nun, auch postoperativ, füll- und entleerbar, um unter Beachtung der Möglichkeiten und Grenzen der Ciliarmuskelaktivität eine, auch später korrigierbare Grundeinstellung der Position der Linse oder des Linsensystems im Auge zu bewirken.

Beispielsweise kann durch eine Mikroinjektion bezüglich des Fluiddepots Flüssigkeit entnommen, aber auch zugeführt werden mit der Folge, dass sich der Füllungsgrad und damit die Grundform des Implantatkörpers selbst verändert, respektive justieren lässt.

Obwohl der Implantatkörper großflächig auf dem Ciliarmuskel aufsitzt, wird eine Unterbindung der Kammerwasserproduktion ausgeschlossen, und zwar durch die offene Struktur der Muskelauflage einerseits und die sichere Verankerung mit Krallen und Hapten andererseits. Im Vergleich zum Stand der Technik, bei dem von einer Verankerung des Linsenimplantats in der Linsenkapsel ausgegangen wird, ist Voraussetzung, dass die Kapsel selbst intakt und für eine entsprechende Implantation geeignet ist. Selbst dann, wenn diese Voraussetzungen erfüllt werden, verbleibt ein großes Problem im Verlauf der Tragezeit. Überwiegend tritt postoperativ nach einigen Monaten ein Nachstar auf. Dieser wird durch eine Eintrübung der Kapselreste verursacht. Die derzeit bekannte Abhilfe besteht in einer Laserbehandlung, wobei die eingetrübten Kapselanteile zerstört werden. Spätestens aber zu diesem Zeitpunkt ist die Linsenkapsel endgültig zerstört und demnach auch das Implantat funktionslos, wobei im problematischsten Fall das Implantat sogar aus der Kapsel wegwandern kann.

Erfindungsgemäß kann grundsätzlich zwar die Linsenkapsel belassen werden, jedoch ist aber ein teilweises oder vollständiges Entfernen, auch nachträglich möglich, und zwar ohne dass die Funktion des Implantats durch das unmittelbare Aufsetzen auf den Ciliarmuskel beeinträchtigt wird.

Die Erfindung soll nachstehend anhand von Figuren näher erläutert werden.

Hierbei zeigen:
- Fig. 1: eine prinzipielle Querschnittsdarstellung bei entfernter Augenlinse;
- Fig. 2: eine Darstellung mit eingesetztem Implantat, entspanntem Ciliar- muskel, Fernsicht und
- Fig. 3: eine Darstellung mit Linsenimplantat bei angespanntem Ciliar- muskel, Nahsicht;

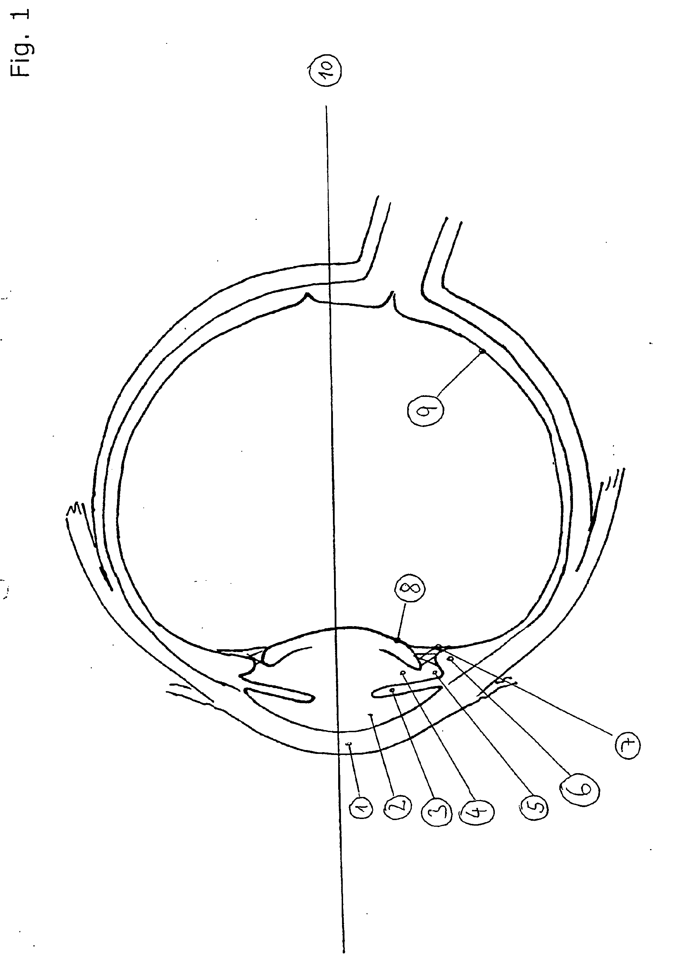

Bei der Darstellung nach Fig. 1 ist im vorderen Bereich des Augapfels die Hornhaut 1 mit dahinter befindlicher vorderer Augenkammer 2 gezeigt. Bezugszeichen 3 definiert die Iris des Auges.

Die Sehachse ist mit dem Bezugszeichen 10 versehen. 5 bezeichnet den Sulcus zwischen den Ciliarmuskeln und der Iris 6, den Ciliarmuskelansatz mit Ciliarmuskel. Die Zonulafasern sind mit dem Bezugszeichen 7, die Linsenkapsel mit dem Bezugszeichen 8 und die Netzhaut mit dem Bezugszeichen 9 versehen.

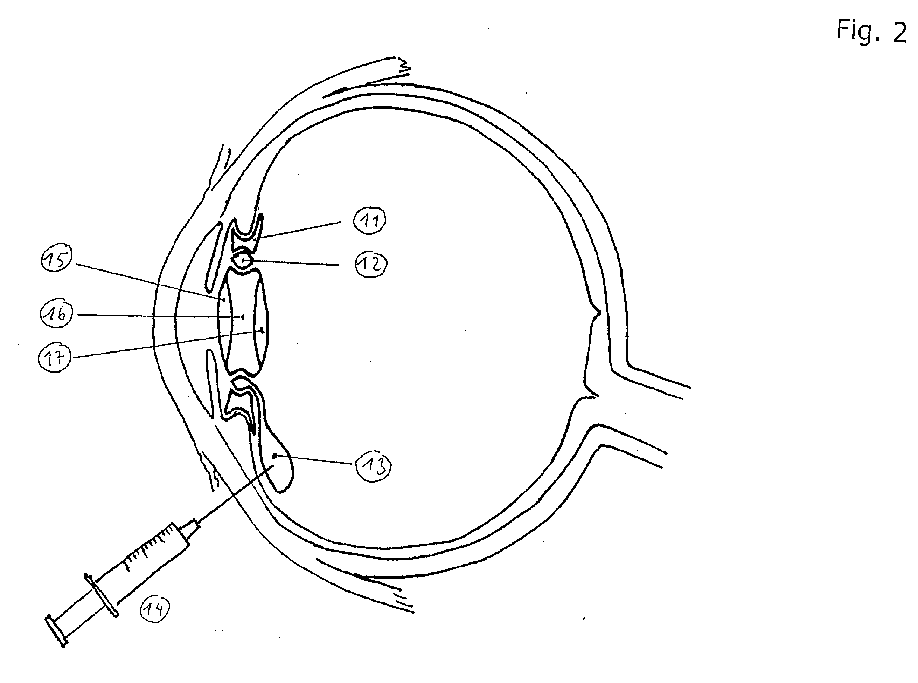

Gemäß der Darstellung nach Fig. 2 ist der Abstandshalter bzw. das intermobile Element 11 erkennbar, welches das Implantat, bestehend aus äußerer Linse 15, fluidgefülltem Hohlraum 16 und innerer Linse 17 fixiert. Zwischen dem intermobilen Element 11 und dem Implantat ist noch ein Fluiddepot 12 in ringförmiger Ausführung vorhanden, das mit einer externen Injektionskammer 13 zum Ausführen einer Fluidinjektion über beispielsweise eine Spritze 14 in Verbindung steht. Gemäß der Fig. 2 befindet sich das Linsenimplantat in einem Zustand bei entspanntem Ciliarmuskel mit Einstellung auf Fernsicht. Die gezeigte Ausführungsform geht von einer dreiteiligen Anordnung mit Linsensystem, Fluidring und externer Injektionskammer zur Justierung und dem erwähnten intermobilen Element bzw. Abstandshalter aus.

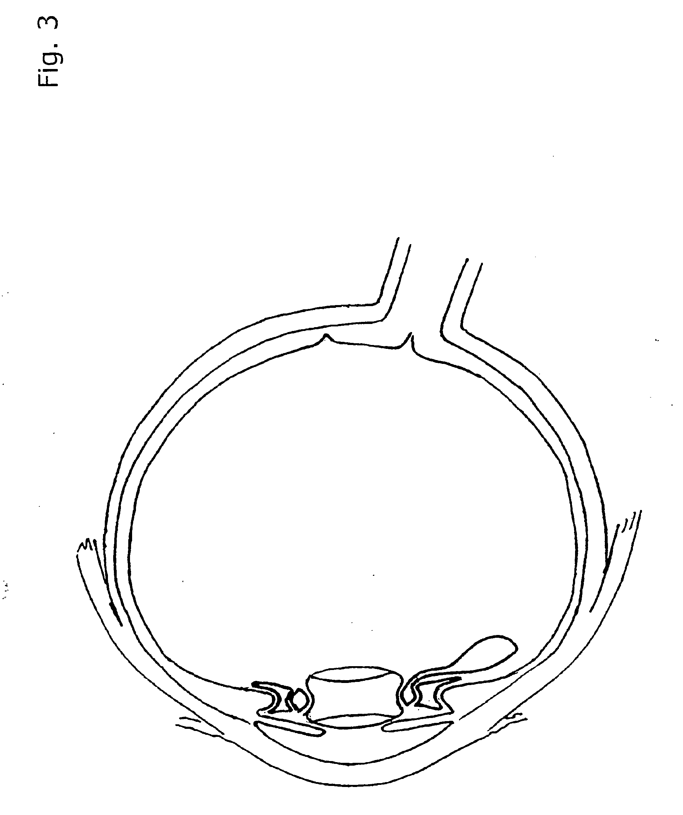

Fig. 3 zeigt das Linsenimplantat in situ bei angespanntem Ciliarmuskel für Nahsicht.

Der Implantatkörper umfasst also eine äußere Linse 15 und eine innere Linse 17. Der Implantatkörper selbst bildet einen Hohlraum 16, der mit einem Fluid, d.h. einer Flüssigkeit oder einem Gas, gefüllt ist.

Über den Ciliarmuskel 6 kann auf den Implantatkörper eine Kraft ausgeübt werden, die dazu führt, den Abstand des oder der Linsen 15, 17 bezogen auf die Netzhaut 9 zu verändern.

Der Hohlraum 16 des Implantatkörpers steht wie erwähnt weiterhin mit dem Fluiddepot 13 in Verbindung, welches bevorzugt im vorderen Teil des Bulbus oculi befindlich ist.

Das Fluiddepot 13 ist postoperativ füll- und entleerbar, um unter Beachtung der Ciliarmuskelaktivität und dem patientenspezifischen Akkommodationsvermögen eine, auch korrigierbare Grundeinstellung der Position der Linsen bzw. des Linsensystems 15, 17 zu bewirken. Der Füllungsgrad des Fluiddepots 12; 13 und damit mittelbar des Hohlraums 16 im Implantatkörper kann über eine Mikroinjektion in dem gewünschten Maße verändert werden, wodurch auch die Position und der Sitz des Implantatkörpers im Sulcus der hinteren Augenkammer optimierbar ist.

Bei einer weiteren Ausführungsform der Erfindung besteht die Möglichkeit, eine für den Arzt sichtbare Markierung der Position des Fluiddepots, durch beispielsweise einen Punkt auf der vorderen Linse vorzusehen.

Ebenso ist es möglich, über das Implantat eine Korrektur von zusätzlichen Sehfehlern vorzunehmen, die üblicherweise mit einer Brille behandelt werden müssen. Gedacht ist hier beispielsweise an Astigmatismus und leichte Formen des Strabismus. Dies wird dadurch möglich, dass die Lage des Implantatkörpers, z.B. unter Zuhilfenahme von Markierungen definiert veränderbar ist und grundsätzlich die Möglichkeit besteht, eine Linse des Linsensystems individuell zu fertigen, bevor diese mit dem Implantatkörper verdrehsicher verbunden wird.

In einer bevorzugten Ausgestaltung der Erfindung wird zur Vermeidung eines unerwünschten Anstiegs des Augeninnendrucks außenumfangsseitig des Implantatkörpers diesem eine Form verliehen, dass das von den Ciliarfortsätzen der hinteren Augenkammer gebildete Kammerwasser in der vorderen Augenkammer rückresorbierbar ist. Hierfür weist der Implantatkörper Öffnungen oder Rinnen auf oder die Kammer des Implantatkörpers zur Aufnahme des Linsensystems wird im Durchmesser reduziert ausgeführt und es wird zwischen Ciliarmuskel und Linsensystem bzw. Implantatkörper ein Abstandshalter oder Zwischenkörper als intermobiles Element eingefügt, welcher einerseits den erforderlichen Muskeldruck weiterleiten kann, andererseits aber die Möglichkeit schafft, dass das Kammerwasser abfließen kann. Der Abstandshalter und der Implantatkörper kann einstückig, d. h. integral ausgeführt sein. Eine mehrteilige Ausführung begünstigt ein minimalinvasiv-chirurgisches Vorgehen, weil kleinere Teile durch den chirurgischen Zugang eingeführt werden müssen.

Zur Verbesserung der Verankerung des Implantatkörpers bezüglich dem Ciliarsulcus können Ausformungen, Krallen oder dergleichen kraft- und/oder formschlüssige Mittel vorgesehen sein.

Um eine minimalinvasive Operation zu gewährleisten, besteht bei einer weiteren Ausführungsform der Erfindung die Möglichkeit, den Implantatkörper mit Linsensystem verformbar bzw. faltbar zu gestalten, wobei der Implantatkörper erst mit dem Einsetzen seine endgültige Form erhält. Diese Formgebung ist beispielsweise durch Aufquellen und/oder durch eine Flüssigkeitsfüllung realisierbar. Eine weitere Möglichkeit der Volumenreduktion bei der Implantation ist das teilweise oder vollständige Entfernen des Fluids aus dem Linsenimplantat.

Zur Übertragung der Ciliarmuskelbewegung ist auch bei dieser Ausführungsform ein Einsatz- oder Distanzstück vorgesehen, welches zur endgültigen Fixage des Linsensystems dient und die gewünschte Übertragung der Ciliarmuskelbewegung gewährleistet.

Das bereits erwähnte Fluiddepot zur Feinjustage kann als außenumfangsseitig des Linsensystems vorgesehener, in einer Ausführungsform elastischer, eine Rückstellkraft aufweisender Schlauchring ausgeführt werden. Dieser Schlauchring kann mit einer Depot-Flüssigkeitsblase für den Fall verbunden sein, dass größere Flüssigkeitsmengen, auch zum Zwecke der nachträglichen Justage, erforderlich sind. Der Distanzring erhält seine endgültige Form erst durch das Fluiddepot und das eingesetzte Linsensystem und besteht beispielsweise aus Einzelsegmenten, die analog einer Kette mit Einzelgliedern zusammengefügt oder durch einen Faden zusammengehalten sind. Auch ein aus einer Drahtspirale gefertigter Ring ist möglich.

In dem Fall, in dem eine exakte Vorausberechnung und Fertigung des Implantatkörpers möglich ist und die Justierung mittels Fluiddepot entfallen kann, besteht die Möglichkeit, den Distanzring, welcher kraftübertragend und als Abstandshalter wirkt, so auszubilden, dass dieser die Linsen des Systems ausreichend fixiert.

Eine operative Technik stellt sich beispielsweise wie folgt dar. Zunächst wird die vordere Augenkammer einige Millimeter weit geöffnet. Im Anschluss erfolgt eine Öffnung der Linsenkapsel im vorderen Teil, um die beschädigte Linse zu entfernen. Der Distanzring, gegebenenfalls in Verbindung mit dem Fluiddepot, wird zusammengelegt und durch den geschaffenen operativen Zugang eingeführt. Von der vorderen Augenkammer wird nun der Ring in die hintere Augenkammer verlegt. Durch die noch vorhandene Linsenkapsel mit Faserapparat wird der Ring selbständig in den Sulcus geführt. Beim Entfalten werden die auf dem Ciliarmuskelring inserierenden Zonulafasern durchtrennt. Es verbleibt allerdings ein Rest von intakten Fasern, welche die verbleibende Linsenkapsel halten.

Anschließend wird nun das Linsensystem ebenso über die vordere in die hintere Augenkammer verlegt. Der Abstandshalter oder Distanzring ist bei dieser Ausführungsform so gestaltet, dass das Linsensystem einrastet. Der Durchmesser ist vorzugsweise hier kleiner als die maximale Irisöffnung.

Zusammenfassend schafft die Erfindung eine Möglichkeit zur Wiederherstellung der Akkomodationsfähigkeit im Rahmen der Entfernung einer natürlichen Augenlinse. Leichte Sehfehler können mit therapiert werden.

In Verbindung mit dem Fluiddepot ist eine Justierung auch postoperativ möglich. Die Erfindung ist unabhängig vom Vorhandensein von Linsenkapsel, Zonulafasern und Glaskörper.

### Bezugszeichenliste

- 1: Hornhaut
- 2: vordere Augenkammer
- 3: Iris
- 4: hintere Augenkammer
- 5: Sulcus zw. proc. ciliaris und Iris
- 6: proc. ciliaris mit Ciliarmuskel
- 7: Zonulafasern
- 8: Linsenkapsel
- 9: Netzhaut
- 10: Sehachse
- 11: intermobiles Element bzw. Abstandshalter
- 12: Fluiddepot in ringförmiger Ausführung
- 13: externe Injektionskammer
- 14: Fluidinjektion
- 15: äußere Linse
- 16: fluidgefüllter Hohlraum
- 17: innere Linse

## Patentansprüche

1. Ciliarmuskel-betätigtes, akkommodationsfähiges Linsenimplantat, bestehend aus mindestens einer oder mehreren auf einer gemeinsamen optischen Achse liegenden Linsen (15; 17) aus einem biokompatiblen Material, wobei die mindestens eine Linse (15; 17) Bestandteil eines flexiblen, geschlossenen, im Bereich der eigentlichen Linse transparenten Implantatkörpers ist, an dessen Außenumfangsfläche der Ciliarmuskel (6) über ein intermobiles Element (11) angreift und dessen Hauptachse auf der Sehachse (10) verläuft,
weiterhin zwischen dem Implantatkörper und dem intermobilen Element (11) ein Fluiddepot (12) vorhanden ist, wobei die axiale Position der Linse oder des Linsensystems (15; 17) über ein Aktivieren des Ciliarmuskels veränderbar ist und das intermobile Element (11) am Ciliarmuskel (6) befestigbar ist sowie einen Kammerwasserdurchtritt ermöglicht,
**dadurch gekennzeichnet, dass**
das Fluiddepot (12) eine Ringform aufweist und mit einer Fluidinjektionskammer (13) in Verbindung steht, welche postoperativ füll- und entleerbar ist, um unter Beachtung der Ciliarmuskelaktivität und dem patientenspezifischen Akkommodationsvermögen eine, auch korrigierbare Grundeinstellung der Position der Linse oder des Linsensystems (15; 17) im Auge zu bewirken.

2. Ciliarmuskel-betätigtes, akkommodationsfähiges Linsenimplantat nach Anspruch 1 ,
**dadurch gekennzeichnet, dass**
der Implantatkörper Öffnungen oder Rinnen zum Durchtritt von Kammerwasser aufweist.

3. Ciliarmuskel-betätigtes, akkommodationsfähiges Linsenimplantat nach einem der vorangegangenen Ansprüche,
**dadurch gekennzeichnet, dass**
das intermobile Element (11) ein aus Einzelsegmenten bestehender Distanzring ist.

## Claims

1. An accommodative lens implant controlled by the ciliary muscle, consisting of at least one or more lenses (15; 17) made of a biocompatible material and disposed on a common optical axis, wherein the at least one lens (15; 17) is a component of a flexible, closed implant body that is transparent in the region of the actual lens, to the outer circumferential surface of which the ciliary muscle (6) is connected by an intermobile element (11) and whose main axis extends on the visual axis (10);
wherein further a fluid depot (12) is provided between the implant body and the intermobile element (11), wherein the axial position of the lens or lens system (15; 17) can be altered by activating the ciliary muscle and the intermobile element (11) is attachable to the ciliary muscle (6) and enables passage of aqueous humor,
**characterized in that**
the fluid depot (12) has an annular shape and is in communication with a fluid-injection chamber (13) that can be filled and emptied postoperatively, so as to achieve a basic setting of the position of the lens or the lens system (15; 17) in the eye that takes into account the ciliary muscle activity and the accommodation ability specific to the patient and that is also correctable.

2. Ciliary-muscle-controlled, accommodative lens implant according to claim 1,
**characterized in that**
the implant body comprises openings or grooves for the passage of aqueous humor.

3. Ciliary-muscle-controlled, accommodative lens implant according to one of the preceding claims,
**characterized in that**
the intermobile element (11) is a spacer ring consisting of individual segments.

## Revendications

1. Implant cristallinien actionné par le muscle ciliaire et apte à l'accommodation, comprenant au moins une ou plusieurs lentilles (15 ; 17) disposées sur un axe optique commun, en un matériau biocompatible, dans lequel ladite au moins une lentille (15 ; 17) est un élément constitutif d'un corps d'implant flexible fermé et transparent dans la région de la lentille proprement dite, tel que le muscle ciliaire attaque la surface périphérique extérieure du corps d'implant via un élément intermédiaire mobile (11), et dont l'axe principal s'étend sur l'axe de vision (10),
il est en outre prévu un dépôt de fluide (12) entre le corps d'implant et l'élément intermédiaire mobile (11), la position axiale de la lentille ou du système de lentilles (15 ; 17) étant modifiable via une activation du muscle ciliaire et l'élément intermédiaire mobile (11) étant susceptible d'être fixé sur le muscle ciliaire (6) et permet une traversée de l'humeur aqueuse,
**caractérisé en ce que**
le dépôt de fluide (12) présente une forme annulaire et communique avec une chambre d'injection de fluide (13), laquelle peut être remplie et vidée de manière postopératoire, afin d'entraîner un réglage de base, capable d'être corrigé, de la position de la lentille ou du système de lentilles (15 ; 17) dans l'oeil, en tenant compte de l'activité du muscle ciliaire et des capacités d'accommodation spécifiques au patient.

2. Implant cristallinien actionné par le muscle ciliaire et apte à l'accommodation selon la revendication 1,
**caractérisé en ce que** le corps d'un implant comporte des ouvertures ou des goulottes pour la traversée de l'humeur aqueuse.

3. Implant cristallinien actionné par le muscle ciliaire et apte à l'accommodation selon l'une des revendications précédentes, **caractérisé en ce que** l'élément intermédiaire mobile (11) est une bague d'écartement constituée de segments individuels.
